# EUROPÄISCHE PATENTSCHRIFT

(11) **EP 0 849 173 B1**
(45) Veröffentlichungstag und Bekanntmachung des Hinweises auf die Patenterteilung: **16.04.2003**
(21) Anmeldenummer: 97121293.1
(22) Anmeldetag: 04.12.1997
(51) Int. Cl.: B65B 3/00, B65B 55/02

(54) **Verfahren zum Herstellen eines befüllten Kunststoff-Spritzenkorpus für medizinische Zwecke**
Method for fabricating a filled plastic syringe body for medical purposes
Procédé pour fabriquer un corps de seringue rempli en matière plastique pour usage médical

(30) Priorität: 18.12.1996 DE 19652708
(43) Veröffentlichungstag der Anmeldung: 24.06.1998
(73) Patentinhaber: Schott Glas, 55122 Mainz (DE); CARL-ZEISS-STIFTUNG trading as SCHOTT GLAS, 55122 Mainz (DE)
(72) Erfinder: Reinhard, Michael, Dr., 55270 Ober-Olm (DE); Bouffleur, Ralf, 55543 Bad Kreuznach (DE); Spallek, Michael, Dr., 55218 Ingelheim (DE)
(74) Vertreter: Fuchs Mehler Weiss & Fritzsche

(56) Entgegenhaltungen:
- EP-A- 0 709 105
- WO-A-94/13541
- WO-A-95/12482
- WO-A-96/13289
- US-A- 4 628 969

## Beschreibung

Die Erfindung bezieht sich auf ein Verfahren zum Herstellen eines befüllten Kunststoff-Spritzenkorpus für medizinische Zwecke, bestehend aus einem Spritzenzylinder, an dessen einem, rückwärtigen Ende, dieses verschließend, ein Kolbenstopfen eingeführt ist, sowie an dessen anderem, austrittsseitigen Ende ein Kopf angeformt ist, der zur Aufnahme eines Applizierelementes ausgebildet ist, und der im Lagerungszustand mittels eines abnehmbaren Verschlußelementes dicht verschlossen ist.

Ein solcher Spritzenkorpus kann beispielsweise ein Spritzenkörper für eine befüllte Kunststoff-Einmalspritze, auch Fertigspritze genannt, sein, wie er in seinen Abmessungen in den Normen DIN 13 098 für ungefüllte Kunststoff-Einmalspritzen sowie in DIN ISO 11 040, Teil 4, für vorgefüllte Glasspritzen festgelegt ist. Der Spritzenkörper für diese Fertigspritzen besteht aus einem Spritzenzylinder, der an seinem Ende, das mit dem Kolbenstopfen verschlossen ist, eine Fingerauflage aufweist, sowie an dessen austrittsseitigen Ende ein Spritzenkopf angeformt ist.

Ein solcher Spritzenkorpus kann auch ein Spritzenzylinder sein, der prinzipiell wie der Spritzenkörper für eine Fertigspritze, jedoch ohne Fingerauflage, ausgebildet ist. Ein solcher Spritzenzylinder wird im folgenden als Spritzenampulle bezeichnet.

Ein solcher Spritzenkorpus kann ferner auch eine Karpule sein, wie sie hinsichtlich ihrer Konfiguration in den Normen DIN ISO 11 040, Teile 1-3 und DIN ISO 13926-1 für Karpulen mit Glaszylinder festgelegt sind.

Diese Karpulen gehören, wie auch die vorgenannten Normen zeigen, generell zu der Gattung der "vorgefüllten Spritzen". Sie bestehen aus einem Spritzenzylinder, dem Karpulzylinder, mit austrittsseitig angeformtem Halsteil, das einen flachen Mündungsrand und einen Randwulst aufweist und mittels einer Aluminium-Bördelkappe mit eingelegter Dichtscheibe dicht verschließbar ist, während das andere Ende des Zylinders mit einem Kolbenstopfen verschließbar ist.

Kunststoff-Fertigspritzen der vorgenannten Art, insbesondere kleinvolumige Fertigspritzen mit einem Volumen vorzugsweise im Bereich von 1-10 ml, werden üblicherweise dergestalt gefertigt, daß der Spritzenkörper als solcher in einem Kunststoffe zu Pharmaverpackungen verarbeitenden Betrieb hergestellt wird, die so hergestellten Spritzenkörper dann, gegebenenfalls nach einer Zwischenlagerung, zum Versand an einen pharmazeutischen Betrieb gelangen, wo sie applizierbereit gefüllt und dicht verschlossen werden.

Die Herstellung der Spritzenkörper als solche ist beispielsweise durch die WO 95/12482 sowie durch die DE 44 38 360 A1 bekanntgeworden. Danach wird zunächst, zumindest unter (partikelarmen) Reinraumbedingungen, der Spritzenkörper durch Spritzgießen hergestellt, gegebenenfalls seine Innenwandung silikonisiert, und der Spritzenkopf durch eine aus gummiartigem Material hergestellte Verschlußkappe, auch als "Tip-Cap" bezeichnet, verschlossen. Am Ende der Herstellung erfolgt in aller Regel ein partikel- und bakteriendichtes Verpacken der so hergestellten Spritzenkörper. Daran schließt sich ein Sterilisationsvorgang an, der beim Einsatz von Gammastrahlen zur Sterilisation meist bei einem anderen Unternehmen durchgeführt wird. Sie stehen dann für eine Zwischenlagerung bzw. für den Versand an den abfüllenden Betrieb bereit. Der sich später daran beim Pharmazeuten anschließende Abfüllvorgang mit dem dichten Verschließen der Fertigspritze läßt sich beispielsweise aus dem Aufsatz von H. Dollinger "Abfüllen von Einmalspritzen in einem Hochleistungs-Kompaktsystem", veröffentlicht in der Zeitschrift: Die Pharmazeutische Industrie, 56, Nr. 1 (1994), in dem das Befüllen von Glas-Fertigspritzen beschrieben wird, sinngemäß entnehmen. Typische Verfahrensschritte sind dabei die Entnahme der angelieferten Spritzenkörper, gegebenenfalls das Reinigen, Trocknen und Sterilisieren, falls die Spritzenkörper nicht sterilisiert ausgeliefert werden, das Füllen und Verschließen der Spritzenkörper, in den meisten Fällen in Verbindung mit dem Sterilisieren des befüllten Spritzenkörpers, sowie das Etikettieren und eine weitere Konfektionierung für den Versand an die Verbraucher.

In analoger Weise verläuft das bekannte Herstellen und Befüllen der eingangs beschriebenen Kunststoff-Spritzenampullen und Kunststoff-Karpulen.

Die beschriebenen Schnittstellen zwischen dem Hersteller des Packmittels aus Kunststoff (kunststoffverarbeitender Betrieb), dem Betrieb der die Sterilisation des leeren Packmittels durchführt, und dem abfüllenden pharmazeutischen Betrieb ist aus den unterschiedlichsten Gründen nachteilig. Maßgebende Gründe sind die zusätzlich notwendigen Schritte des Transports, des Ein- und Auspackens der Spritzenelemente, deren Vereinzelung, der Qualitätssicherung sowie die zusätzlichen partikularen und mikrobiologischen Kontaminierungsgefahren bei der Lagerung und dem Transport der ungefüllten Spritzenzylinder für den jeweiligen Spritzenkorpustyp. So lädt sich beispielsweise der Spritzenkorpus beim Transport statisch auf, d. h. es haftet Staub an ihm, der vor dem Verfüllen beseitigt werden muß.

Auch ist der mit der Reinigung/Trocknung und Sterilisation entstehende schnittstellenbedingte Aufwand nicht unerheblich. Der Aufwand ist schon bei einem Glas-Spritzenkörper gemäß dem vorgenannten Stand der Technik sehr hoch, obwohl sich Glaskörper - im Gegensatz zu Kunststoff-Körper - noch auf relativ einfache Weise durch Anwendung hoher Temperaturen trocknen lassen. Bei Kunststoffkörpern ist er noch sehr viel aufwendiger. Diese aufwendigen Prozeduren gehen beispielsweise aus der EP 0 227 401 hervor, in der auch ein spezieller Waschprozeß definiert wird. Ferner ist zu bedenken, daß für das Spritzgießen von Spritzenkörpern ein innerer Kern notwendig ist, durch den es zu einem intensiven Kontakt der heißen Polymerschmelze und beim Herausziehen dieses Kernes zu einem Reibungsvorgang auf der kompletten Innenfläche, der wiederum Abriebartikel etc. erzeugt kommt, was wiederum die in der vorgenannten EP-Schrift beschriebenen aufwendigen Wasch- und Depyrogenisierungsprozesse notwendig macht.

Es ist auch in der Verpackungstechnik bekannt, unter kontrollierten Umgebungsbedingungen in einer einzigen, geschlossenen, d.h. durchgängig automatisierten Fertigungslinie sowohl den Verpackungskörper herzustellen als auch diesen zu befüllen und applizierbereit zu verschließen.

Ein derartiges Verfahren, das unter dem schlagwortartigen Begriff "blow-fillseal" bekanntgeworden ist, wird beispielsweise in den US-Patentschriften 4 671 763, 3 919 374 und 4 995 511 beschrieben. Bei diesem Verfahren wird aufgeschmolzenes Kunststoff-Granulat durch Extrusionsblasen in eine Form eingeblasen und so der Pharmaverpackungskörper hergestellt, der, noch in der Form befindlich, befüllt und anschließend dicht verschlossen wird.

Mit diesem Blas-Verfahren können jedoch nur weiche Kunststoff-Materialien, wie z.B. Polypropylen und Polyethylen, bei niedrigen Temperaturen verarbeitet werden. Es können dabei nur Behälter gefertigt werden, deren Innenmaße relativ grosse Toleranzen aufweisen dürfen, da ein das Innenmaß mit engen Toleranzen vorgebbarer Kern beim Blasverfahren nicht anwendbar ist. So werden typischerweise mit dem bekannten Blasverfahren flaschenähnliche Behälter hergestellt, die sofort nach der Ausformung, d.h. in noch heißem Zustand, befüllt und verschlossen werden. Die Toleranzen der Innenmaße solcher Behälter sind recht groß, jedoch für die Funktionstüchtigkeit des Behälters nicht entscheidend.

Mit dem bekannten Blas-Verfahren sind auch keine starrwandigen pharmazeutischen Primärpackmittel, wie befüllte Kunststoff-Spritzen oder Fläschchen, herstellbar, weil diese Art der Pharmaverpackung ein formfestes Kunststoffmaterial und geringe Toleranzen des inneren Behälterdurchmessers benötigt, um eine ausreichende Abdichtung während der typischerweise mehrjährigen Lagerzeit der befüllten Spritzen bzw. Fläschchen zu gewährleisten.

Der Erfindung liegt die Aufgabe zugrunde, das eingangs bezeichnete Verfahren zur Herstellung eines befüllten Kunststoff-Spritzenkorpus so zu führen, daß es auf einfache Weise unter Minimierung von Logistikkosten sowie unter Verbesserung der Silikonisierung des Kunststoff-Spritzenkorpus durchführbar ist.

Die Lösung dieser Aufgabe gelingt erfindungsgemäß mit den innerhalb einer einzigen, durchgängigen Fertigungslinie in einer kontrollierten Umgebung ausgeführten Verfahrensschritten:
- Fertigen des Kunststoff-Spritzenkorpus durch Spritzgießen in einer entsprechenden formgebenden Höhlung des Spritzwerkzeuges mit Vorgabe des Innenmaßes durch einen Kern,
- Verschließen des einen Endes des Kunststoff-Spritzenzylinders,
- Silikonisieren der vom Spritzgießen noch warmen und aktiven Oberfläche der Innenwandung des Kunststoff-Spritzenzylinders, alternativ auch vor dem Verschließen seines einen Endes,
- Befüllen des Kunststoff-Spritzenkorpus mit dem Spritzeninhalt über das andere, offene Ende des Kunststoff-Spritzenzylinders,
- Verschließen des anderen Endes des Spritzenzylinders.

Durch das erfindungsgemäße Verfahren mit "Spritzgießen-Füllen- und Verschließen" in einer durchgängigen Fertigungslinie, in Analogie zu dem vorne beschriebenen "blow-fill-seal"-Verfahren kurz auch als "injection moldfill-seal"-Verfahren bezeichnet, läßt sich der eingangs bezeichnete befüllte Kunststoff-Spritzenkorpus, der ja ein Massenprodukt darstellt, auf besonders einfache Weise unter Minimierung von Logistik-Kosten und damit kostengünstig, in einem Zuge, d.h. direkt, applizierbereit herstellen.

Besonders vorteilhaft ist es auch bei der direkten Befüllung des Spritzenkorpus, daß die notwendigerweise aufzutragende Gleitmittelschicht (typischerweise Silikonöl) direkt auf die vom Spritzgußvorgang noch warme und aktive Oberfläche aufgebracht werden kann. Dies führt zu einer verbesserten Oberflächenhaftung des Gleitmittels und somit zur deutlich geringeren Abgabe von Gleitmittel oder Gleitmittelbestandteilen an die Lösung während der Lagerung.

Diese Maßnahme ist nur bei einer In-line-Fertigung möglich, da bei einer Befüllung "außer Haus" sich der Spritzenkorpus statisch aufladen und sich Staub an der silikonisierten Oberfläche niederschlagen würde, der beim Pharmazeuten praktisch nicht mehr zu beseitigen wäre.

Bei der erfindungsgemäßen In-line-Fertigung kann man auch die Partikel maßgebend reduzieren durch Ausblasen des Innenraumes des Spritzenkorpus mit ionisierter, partikelfreier Luft, die auch eine Entladung der Kunststoff-Innenwandung bewirkt. Diese Entladung hält jedoch nur eine bestimmte Zeitdauer vor, ausreichend im Rahmen der In-line-Fertigung, nicht ausreichend bei einer Befüllung "außer Haus".

Hinsichtlich des Herstellens eines Kunststoff-Spritzenkorpus für eine befüllte Einmalspritze (Fertigspritze), bestehend aus einem Kunststoff-Spritzenkörper mit einem Kunststoff-Spritzenzylinder, an dessen rückwärtigem Ende eine Fingerauflage angebracht ist, sowie an dessen austrittsseitigem Kopfende ein Kunststoff-Spritzenkopf mit einem Adapterkonus oder einer verriegelbaren Kegelverbindung zur Aufnahme des Gegenstückes des Applizierelementes angeformt ist, gelingt die Lösung der Aufgabe nach einer Weitergestaltung der Erfindung mit den innerhalb der einzigen, durchgängigen Fertigungslinie in einer kontrollierten Umgebung ausgeführten Verfahrensschritten:
- Fertigen des Kunststoff-Spritzenkörpers durch Spritzgießen in einer entsprechenden formgebenden Höhlung des Spritzgießwerkzeuges mit Vorgabe des Innenmaßes durch einen Kern,
- Anbringen des Verschlusses an dem Spritzenkopf des Kunststoff-Spritzenkörpers,
- Silikonisieren der vom Spritzgießen noch warmen und aktiven Oberfläche der Innenwandung des Kunststoff-Spritzenzylinders, alternativ auch vor dem Anbringen des Verschlusses,
- Befüllen des Kunststoff-Spritzenkörpers mit dem Spritzeninhalt über das rückwärtige offene Ende des Kunststoff-Spritzenzylinders und
- Verschließen des rückwärtigen offenen Endes des Kunststoff-Spritzenzylinders mit dem Kolbenstopfen.

Mit Hilfe dieses Verfahrens lassen sich somit befüllte Einmalspritzen, die Fertigspritzen, auf besonders einfache und damit kostengünstige Weise herstellen.

In entsprechender Weise laufen die Verfahrensschritte gemäß einer weiteren Ausgestaltung der Erfindung bei der Herstellung der eingangs bezeichneten Spritzenampullen ab.

Hinsichtlich des Herstellens eines Kunststoff-Spritzenkorpus für eine befüllte Karpule, bestehend aus einem Kunststoff-Karpulen-Zylinder als Spritzenzylinder, der rückwärtig mit einem Kolbenstopfen verschlossen ist, und der austrittsseitig ein angeformtes Halsteil mit flachem Mündungsrand und einem Randwulst aufweist, das mittels einer Aluminium-Bördelkappe mit eingelegter Dichtscheibe dicht verschlossen ist, gelingt die Lösung der Aufgabe nach einer weiteren Ausbildung der Erfindung mit den innerhalb der einzigen durchgängigen Fertigungslinie in einer kontrollierten Umgebung ausgeführten Verfahrensschritten:
- Fertigen des Kunststoff-Karpulen-Zylinders durch Spritzgießen in einer entsprechenden formgebenden Höhlung des Spritzgießwerkzeuges mit Vorgabe des Innenmaßes durch einen Kern,
- Verschließen des Halsteiles mit der von außen zugeführten Aluminium-Bördelkappe einschließlich Dichtscheibe,
- Silikonisieren der vom Spritzgießen noch warmen und aktiven Oberfläche der Innenwandung des Kunststoff-Karpulen-Zylinders, alternativ auch vor dem Aufbringen der Aluminium-Bördelkappe mit Dichtscheibe,
- Befüllen des Kunststoff-Karpulen-Zylinders mit dem Spritzeninhalt über das rückwärtige offene Ende des Kunststoff-Karpulen-Zylinders,
- Verschließen des rückwärtigen Endes mit dem Kolbenstopfen.

Mittels dieses erfindungsgemäßen Verfahrens lassen sich somit auch Karpulen besonders kostengünstig herstellen.

In besonderen Fällen kann es günstig sein, den Kunststoff-Spritzenkorpus nicht über das rückwärtige Ende zu befüllen sondern kopfseitig. Das Verfahren wird nach einer weiteren Ausgestaltung dann so durchgeführt, daß zunächst das rückwärtige Ende des Kunststoff-Spritzen- bzw. Karpulen-Zylinders verschlossen, danach dieser Zylinder über das austrittsseitige Kopfende gefüllt und anschließend dieses Kopfende verschlossen wird.

Auch zum Sterilisieren des befüllten Kunststoff-Spritzenkorpus sind verschiedene Möglichkeiten denkbar. Je nach verfüllter Lösung erfolgt das Sterilisieren entweder durch Autoklavierung oder durch eine geeignete energiereiche Strahlung, z.B. Gammastrahlen, Mikrowellen, Betastrahlen, Lichtblitze. Diese Sterilisationsschritte können prinzipiell entfallen, wenn die Verfahrensschritte, einschließlich des Verschließens des befüllten Spritzenkorpus, unter aseptischen Umgebungsbedingungen erfolgen. Nach dem Befüllen und Verschließen und ggf. der gesonderten Sterilisation des Spritzenkorpus erfolgt seine Etikettierung und weitere Konfektionierung.

Das Anbringen des Verschlusses an dem Spritzenkopf des Spritzenkörpers im Falle der Kunststoff-Fertigspritze und der Spritzenampulle kann auf unterschiedliche Weise erfolgen. Hierbei stehen dem Fachmann eine Reihe von Möglichkeiten zur Verfügung. Für die Auswahl sind auch Kundenwünsche bzw. Verbrauchergewohnheiten entscheidungserheblich.

Im einfachsten Fall wird in üblicher Weise eine Verschlußkappe aus gummiartigem Material, ein sog. Tip-Cap, als Verschlußelement verwendet. Das Tip-Cap kann dabei als separates Teil zugeführt werden, es kann aber auch innerhalb des geschlossenen kontrollierten Volumens im Wege des Spritzgießens unmittelbar aufgespritzt werden.

Alternativ kann ein Knebelverschluß aus weichem Kunststoff mit einer Sollabrißstelle angespritzt werden.

Ein besonders günstiges Anbringen des Verschlusses ist erzielbar durch Anformen eines Knebelverschlusses in Form eines Verschlußnippels aus hartem Kunststoffmaterial mit Sollbruchstelle im Wege des Spritzgießens, über den anschließend eine Schutzkappe aus einem weichelastischen Kunststoff angespritzt wird.

Es ist auch eine einfache Lösung denkbar, bei der der Austrittskanal des Spritzenkopfes direkt mittels eines Propfens, vorzugsweise aus Kunststoff, formschlüssig verschlossen wird.

Anhand von in den Zeichnungen dargestellten Ausführungsbeispielen wird die Erfindung näher erläutert. Dabei ergeben sich auch weitere Ausgestaltungen der Erfindung.

Es zeigen:
- Fig. 1: zwei Ausführungsbeispiele eines gemäß dem erfindungsgemäßen Verfahren hergestellten befüllten Spritzenkorpus, nämlich ein Spritzenkörper einer Fertigspritze in Fig. 1a mit einem Luer-Konus und einer Spritzenampulle in Fig. 1b mit einer verriegelbaren Kegelverbindung,
- Fig. 2: eine schematische Darstellung der einzelnen Verfahrensschritte des erfindungsgemäßen Verfahrens für die Herstellung von Fertigspritzen nach Fig. 1a,
- Fig. 3: zwei weitere Ausführungsformen für den Verschluß des Spritzenkopfes einer Fertigspritze bzw. einer Spritzenampulle,
- Fig. 4: eine Ausführungsform für die teilweise Unterbringung der Spritzgießmaschine in einer kontrollierten Umgebung,
- Fig. 5: ein weiteres Ausführungsbeispiel für den befüllten Spritzenkorpus in Form einer Karpule, und
- Fig. 6: eine schematische Darstellung der einzelnen Verfahrensschritte des erfindungsgemäßen Verfahrens zur Herstellung der Karpule nach Fig. 5.

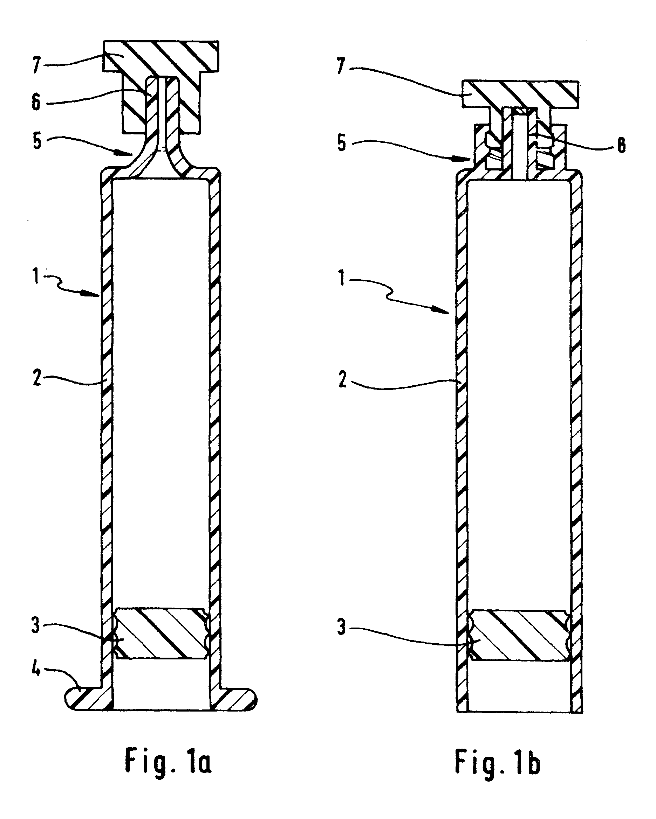

Die in Fig. 1 a dargestellte befüllte Kunststoff-Fertigspritze mit einer Konfiguration nach DIN ISO 11040-4 für Glas besitzt einen Spritzenkörper, der generell mit 1 bezeichnet ist. Dieser Spritzenkörper setzt sich zusammen aus einem Spritzenzylinder 2, der an einem Ende mit einem beweglichen Kolbenstopfen 3 verschlossen ist, an dem in bekannter Weise eine Kolbenstange (nicht dargestellt) beim Applizieren anbringbar ist.

An den Spritzenzylinder 2 in Fig. 1a ist ferner eine übliche Fingerauflage 4 angeformt, wobei die Ausführung auch so getroffen werden kann, daß diese Fingerauflage als gesondertes Teil nachträglich anbringbar ist. Hierzu kann eine (nicht dargestellte) Ringnut am rückwärtigen Ende des Spritzenzylinders angebracht werden, um die Befestigung der Fingerauflage zu vereinfachen. Es können auch formschlüssige Schnappverbindungen mit Hinterschneidungen, ggf. in Verbindung mit einem eingeschobenen Sicherungsring gemäß der DE 4434644 A1, Fig 1b und 1c, vorgesehen sein.

Diese Fingerauflage 4 ist bei dem befüllten Pharmaverpackungskörper nach Fig. 1b nicht vorgesehen, den man daher üblicherweise anstelle des Spritzenkörpers nach Fig. 1a als "Spritzenampulle" bezeichnet, der jedoch im übrigen eine Konfiguration wie der Spritzenkörper nach Fig. 1a hat, weswegen gleiche Teile mit gleichen Bezugszeichen versehen sind.

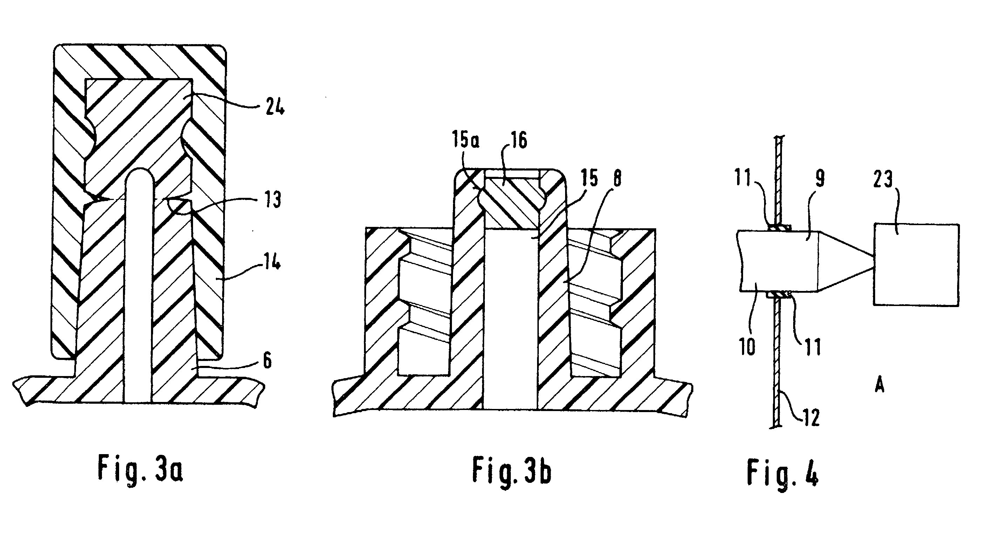

Das andere Ende des Spritzenzylinders 2 ist jeweils mit einem angeformten Spritzenkopf 5 versehen, der bei dem Spritzenkörper nach Fig. 1a in einen sogenannten LUER-Konus 6, und bei der Spritzenampulle nach Fig 1b in eine verriegelbare Kegelverbindung 8 (LUER-LOCK), wie sie auch in Fig. 3b zu erkennen ist, übergeht. An diesem LUER-Konus 6 kann in bekannter Weise im Applizierfall eine Standardnadel mit einem angepaßten Aufsatz (nicht dargestellt) angebracht werden. Entsprechend kann die verriegelbare Kegelverbindung 8 in Fig. 1b mit dem zugehörigen, an einer Infusionsleitung oder dergleichen angebrachten Gegenstück verbunden werden. (Details hierzu sind in der ISO-Norm 594, Teile 1 und 2, beschrieben.)

Der Spritzenkopf 5 ist jeweils mittels eines Verschlußelementes 7 dicht verschlossen. Dargestellt ist in den Fig. 1a und 1b ein Verschlußelement in Form einer separaten, aus einem gummiartigen Material bestehenden, aufgesteckten Verschlußkappe, dem Tip Cap. Alternativ kann auch ein sogenannter Knebelverschluß, der fertigungstechnisch günstig, weil direkt anspritzbar, vorgesehen sein. Es ist auch möglich, einen Verschluß gemäß den Figuren 3a und b vorzusehen, die später noch erläutert werden.

Die vergrößert dargestellte Fertigspritze nach Fig. 1a bzw. die Spritzenampulle nach Fig. 1b, hat im Ausführungsbeispiel vorzugsweise ein Volumen im Bereich 0,5-10 ml und kann mit für medizinische Zwecke geeigneten Lösungsmitteln zum Auflösen pulverförmiger Arzneimitel oder Diagnostika, z.B. Wasser zur Injektion, Kochsalzlösung, Wasser mit bakteriostatischen Zusätzen oder mit medizinisch wirksamen Substanzen gefüllt sein.

Der Spritzenkorpus ist aus einem Kunststoff hergestellt, der generell klar transparent, autoklavier- bzw. wasserdampfsterilisierbar oder sterilisierbar durch energiereiche Strahlung sein muß, und zumindest eine Barriere gegen Wasserdampfdiffusion bilden muß, damit der befüllte Spritzenkorpus langzeitlagerfähig ist, d.h. der Verlust an Wasser und die damit verbundene Konzentrationserhöhung akzeptabel ist. Vorzugsweise wird der Spritzenkorpus aus einem zyklischen Olefin-Copolymer (COC) hergestellt, weil dieser Werkstoff neben der notwendigen mechanischen Festigkeit einerseits eine exzellente Barriere gegen Wasserdampf bietet und gleichzeitig glasklar transparent ist. Es ist auch denkbar, den Spritzenkorpus mehrschichtig mit unterschiedlichen Kunststoffen aufzubauen bzw. gesonderte Sperrschichten einzubringen, so wie es in der DE 44 38 360 A1, auf deren Offenbarung hiermit Bezug genommen wird, beschrieben ist.

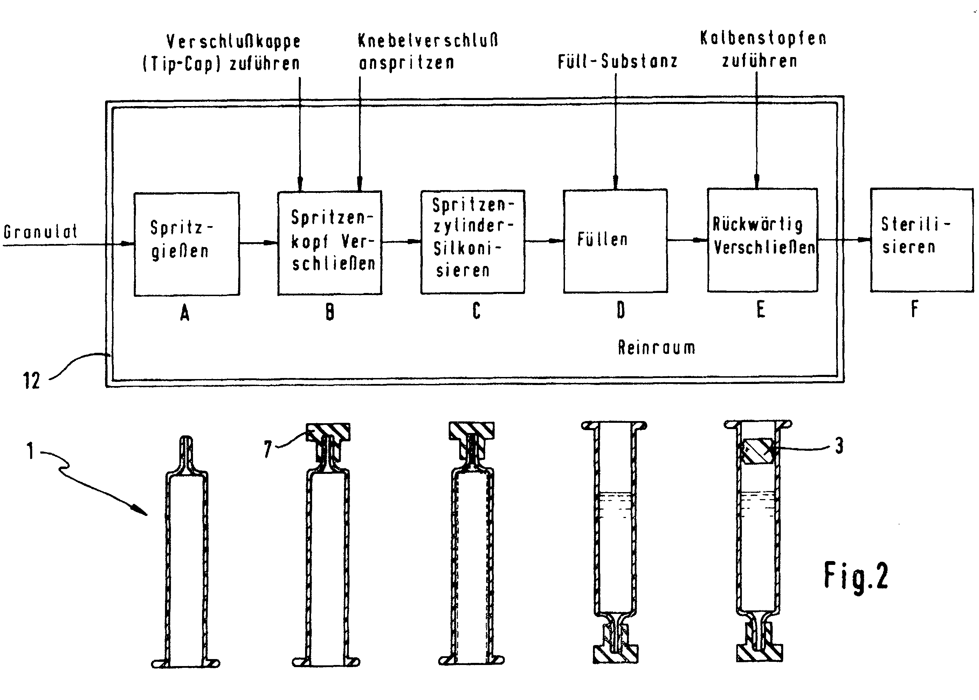

Anhand der Figur 2 soll nunmehr der Verfahrensablauf mit den Verfahrensschritten A-F bei der Herstellung des befüllten Spritzenkörpers einer Fertigspritze nach Figur 1a erläutert werden. Für die Herstellung der Spritzenampulle nach Fig. 1b sind die Verfahrensschritte entsprechend.

Die Verfahrensschritte A-E finden dabei im Ausführungsbeispiel in einem staubfreien, d.h. partikelarmen Raum, einem sog. Reinraum statt, der durch die Doppellinie symbolisch umgrenzt ist. Ist der Reinraum zusätzlich keimarm, spricht man von einem Sterilraum. Es muß jedoch nicht zwingend ein konkreter Raum eines Gebäudes oder eine begehbare Kabine sein. Generell kommt es auf einen abgeschlossenen Bereich mit kontrollierten Umgebungsbedingungen, auf ein kontrolliertes Volumen an. Dieser Bereich kann auch ein sog. Isolator sein, d.h. eine engbegrenzte gekapselte Kammer mit einem kontrollierten kleinen Volumen.

In dem unteren Teil der Fig. 2 sind dabei die einzelnen Verfahrensschritte anhand der schematisch im jeweiligen Fertigungszustand dargestellten Spritze illustrativ gezeigt.

Die Verfahrensschritte A-F finden erfindungsgemäß in einer einzigen, durchgängigen Fertigungslinie statt, so daß externe Schnittstellen vermieden werden.

In der Stufe A - Spritzgießen - wird der Spritzenkörper 1 durch Spritzgießen in einer reinraumfähigen Spritzgießmaschine hergestellt. Dieser Maschine wird von außen in üblicher Weise die granulatförmige Spritzgießmasse zugeführt. Dieses Spritzgießen, bei dem mittels einer Spritzeinheit die Spritzgießmasse plastifiziert und dosiert in eine formgebende Höhlung eines Spritzgießwerkzeuges eingespritzt wird, ist gängiger Stand der Technik und braucht daher nicht näher erläutert zu werden.

Alternativ zu der Ausbildung nach Fig. 2, bei der die gesamte Spritzgießmaschine sich innerhalb des kontrollierten Volumens befindet, kann die Anordnung gemäß Fig. 4 auch so getroffen werden, daß sich nur das formgebende Spritzgießwerkzeug 23 und das düsenseitige Ende 9 der länglichen Spritgießzeinheit in dem kontrollierten Volumen befinden, wobei Dichtelemente 11 in der symbolisch angedeuteten Begrenzung 12 verhindern, daß die Bedingungen im kontrollierten Volumen negativen äußeren Einflüssen ausgesetzt werden. Die in der Spritgießzeinheit 10 herrschende Temperatur zum Plastifizieren der Spritzgießmasse sorgt dabei für die notwendige Keimfreiheit (Sterilität) bzw. Pyrogenfreiheit des geformten Spritzenkörpers.

In der Stufe B - Spritzenkopf Verschließen - wird der Verschluß 7 an dem Spritzenkopf des Spritzenkörpers angebracht. Im Ausführungsbeispiel ist das Aufstecken einer zugeführten ggf. sterilisierten Verschlußkappe (Tip Cap) vorgesehen. Alternativ ist auch das Anformen eines Knebelverschlusses mit Sollabrißstelle aus elastischem oder weichem Kunststoff-Material, beispielsweise Polyethylen, im Wege des Spritzgießens gezeigt. Das Spritzgießen des Knebelverschlusses kann dabei unter Zufuhr eines zweiten Kunststoffmaterials in derselben Spritzgußform erfolgen, in der der Spritzenkörper geformt wird (Stufe A).

Auch das Tip-Cap kann mit einem thermoplastischen Elastomeren direkt auf den Spritzenkopf aufgespritzt werden.

Eine weitere Möglichkeit des Verschließens, eine Ausführungsform mit Originalitätsverschluß, ist in der Fig. 3a gezeigt. In dieser Figur 3a ist in einer gegenüber der Figur 1a vergrößerten Darstellung ausschnittsweise der LUER-Konus 6 gezeigt, der mittels eines Knebelverschlusses in Form eines angespritzen Verschlußnippels 24 aus hartem Kunststoff, z.B. einem COC, mit Sollbruchstelle 13, verschlossen ist. Zur Sicherung der Keimfreiheit des Luer-Konus und des Sollbruchstellen-Bereiches ist zusätzlich eine Abdeck- oder Schutzkappe 14 aus einem weichelastischen Kunststoff, vorzugsweise einem thermoplastischen Elastomer (TPE) angespritzt, die im Applizierfall zusammen mit dem abgebrochenen Verschlußnippel 12 abgedreht wird.

Eine weitere Verschlußmöglichkeit zeigt die Fig. 3b am Beispiel der verriegelbaren Kegelverbindung nach Fig. 1b im vergrößerten Maßstab. Der Verschluß erfolgt durch teilweises Füllen des Austrittskanals 15 des Spritzenkopfes mit einem weichelastischen Kunststoff 16. Über eine Ringnut 15a im Kanal wird dabei eine formschlüssige Verbindung hergestellt. Dieses Verschließen kann entweder durch Spritzgießen eines Elastomers (TPE) erfolgen, oder durch Einsetzen eines vorgefertigten Teiles. Bei der Anwendung wird dieses Verschlußteil 16 nicht entfernt, sondern vielmehr durch eine entsprechende Kanüle mit doppelseitigem Schliff durchstochen.

Ein derartiger Verschluß 16 kann auch im Falle des Spritzenkörpers nach der Fig. 1a vorgesehen sein.

In der Stufe C - Spritzenzylinder Silikonisieren - wird die Innenwandung des Spritzenzylinders mit üblichen Methoden silikonisiert. Der Verfahrensschritt der Stufe C kann auch vor dem der Stufe B durchgeführt werden.

In der Stufe D - Füllen - wird der Spritzenkörper mit der jeweiligen Substanz befüllt, die von außen zugeführt wird.

In der Stufe E - Rückwärtig Verschließen - erfolgt das Verschließen des rückwärtigen offenen Endes des Spritzenzylinders mit dem Kolbenstopfen 3, der vorzugsweise als getrenntes steriles Teil von außen zugeführt wird, der aber auch alternativ durch Spritzgießen unmittelbar in den Spritzenzylinder erzeugbar ist.

In der Stufe F - Sterilisieren - wird die befüllte und komplett verschlossene Fertigspritze einem Sterilisationsvorgang unterworfen. Dies kann bei hitzebeständigem Füllgut auf einfache Weise durch Autoklavieren erfolgen. Bei nicht hitzebeständigem Füllgut erfolgt das Sterilisieren vorzugsweise durch eine energiereiche Strahlung, insbesondere eine Gamma-Strahlung. Auch eine Bestrahlung mit Mikrowellen ist denkbar.

Die Stufe F kann grundsätzlich entfallen, wenn die Verfahrensschritte A-E in einem Sterilraum, d.h. einem zusätzlich keimfreien Reinraum durchgeführt werden. Seine zusätzliche Anwendung erhöht die Sicherheit gegen Keime. Ist der kontrollierte Bereich dabei eine vorne erwähnte gekapselte Kammer, dann kann diese auf einfache Weise mit Heißdampf, H₂O₂ oder anderen einschlägigen Medien sterilisiert werden.

In manchen Fällen kann es vorteilhaft sein, den Spritzenkörper 1 durch die Öffnung im Spritzenkopf zu befüllen, nachdem zuvor das rückwärtige, offene Ende des Spritzenzylinders mit dem Kolbenstopfen verschlossen wurde. Anschließend wird dann der Spritzenkopf mit dem Verschluß 7 versehen. Die entsprechenden Verfahrensschritte der zugehörigen Stufen würden dann in der Reihenfolge E, D und B entsprechend ablaufen.

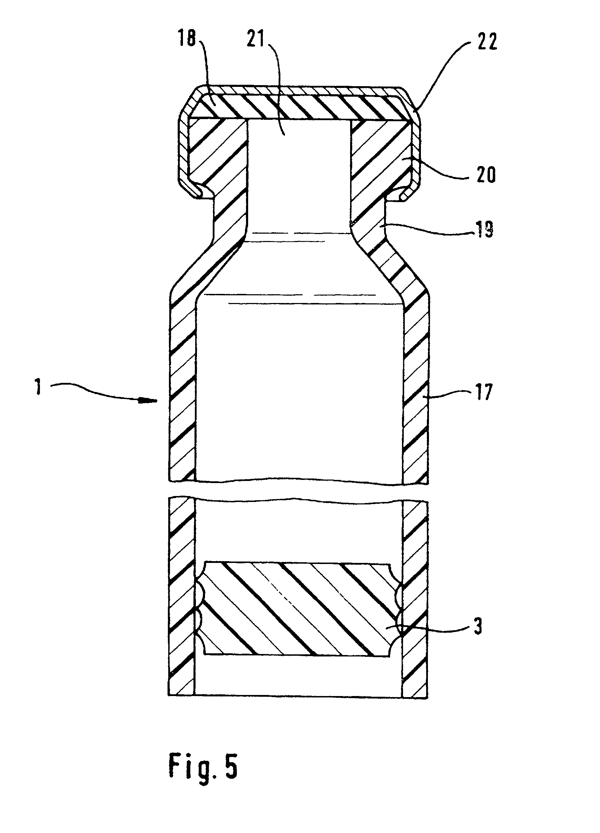

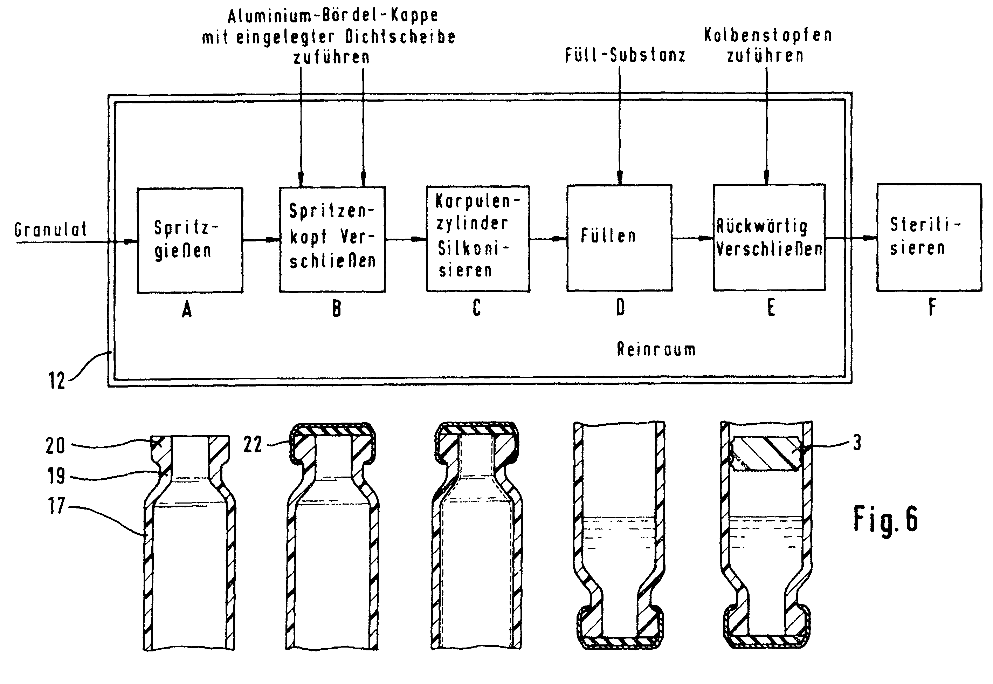

Das Verfahren nach Fig. 2 kann analog für Kunststoff-Spritzenzylinder angewendet werden, die als Karpule gemäß den eingangs zitierten Normen ausgebildet sind. Eine solche befüllte Karpule ist schematisch im vergrößerten Maßstab in der Fig. 5 dargestellt. Das zugehörige Herstellungsverfahren zeigt die Fig. 6.

Die Fig. 5 zeigt eine befüllte Kunststoff-Karpule bestehend aus einem Zylinder 17, der bodenseitig durch einen Kolbenstopfen 3 verschlossen ist. Austrittsseitig weist der Zylinder 17 ein angeformtes Halsteil 19 mit einem Randwulst 20 auf. Die Austrittsöffnung 21 mit einem flachen Mündungsrand ist mittels einer Aluminium-Bördelkappe 22 mit eingelegter Dichtscheibe 18 dicht verschlossen, die auf den Randwulst 20 aufgebördelt ist.

Das zugehörige Herstellungsverfahren zeigt die Fig. 6, die entsprechend der Fig. 2 aufgebaut ist.

In der Stufe A erfolgt das Spritzgießen des Kunststoffzylinders 17 mit Halsteil 19 und Randwulst 20. In der Stufe B erfolgt das Verschließen des Zylinders mit der von außen zugeführten, ggf. sterilisierten Aluminium-Bördelkappe 22 mit Dichtscheibe. Im Schritt C erfolgt das Silikonisieren der Innenwandung des Zylinders 17 der Karpule, an den sich im Schritt D das Befüllen anschließt. Im Schritt E wird der Zylinder mit dem Kolbenstopfen 3, der entweder von außen, ggf. sterilisiert, zugeführt oder die im Reinraum durch Spritzgießen erzeugt wird, verschlossen

Die übrigen Maßnahmen sind wie im Fall der Fig. 2, einschließlich der dort beschriebenen Varianten (Schritt C ggf. vor Schritt B und Ablauf des Füllens und Verschließens ggf. in der Reihenfolge der Schritte E, D und B).

Durch Veränderung des Durchmesser/Längen-Verhältnisses des Spritzenzylinders 17 der Karpule lassen sich Füllvolumina von 1-50 ml und darüber erreichen. Typische Füllvolumina sind 1,5 und 3 ml.

Die befüllten Karpulen-Zylinder 17 können entweder in Spritzengestelle (z.B. zur Anwendung für die Lokalanästesie in der Dentalmedizin) oder in sog. Pen-Systeme (z.B. zur Applikation von Insulin) eingesetzt werden.

## Patentansprüche

1. Verfahren zum Herstellen eines befüllten Kunststoff-Spritzenkorpus (1) für medizinische Zwecke, bestehend aus einem Kunststoff-Spritzenzylinder (2,17), an dessen einem, rückwärtigen Ende, dieses verschließend, ein Kolbenstopfen (3) eingeführt ist, sowie an dessen anderem, austrittsseitigen Ende ein Kopf (5,6,8,19,20) angeformt ist, der zur Aufnahme eines Applizierelementes ausgebildet ist, und der im Lagerungszustand mittels eines Verschlußelementes (7; 12, 14, 16; 22) dicht verschlossen ist, mit den innerhalb einer einzigen durchgängigen Fertigungslinie in einer kontrollierten Umgebung (12) ausgeführten Verfahrensschritten:
- Fertigen des Kunststoff-Spritzenkorpus (1) durch Spritzgießen in einer entsprechenden formgebenden Höhlung des Spritzwerkzeuges mit Vorgabe des Innenmaßes durch einen Kern,
- Verschließen des einen Endes des Kunststoff-Spritzenzylinders (2,17),
- Silikonisieren der vom Spritzgießen noch warmen und aktiven Oberfläche der Innenwandung des Kunststoff-Spritzenzylinders, alternativ auch vor dem Verschließen seines einen Endes,
- Befüllen des Kunststoff-Spritzenkorpus (1) mit dem Spritzeninhalt über das andere, offene Ende des Kunststoff-Spritzenzylinders,
- Verschließen des anderen Endes des Kunststoff-Spritzenzylinders (2,17).

2. Verfahren zum Herstellen eines Kunststoff-Spritzenkorpus für eine befüllte Einmalspritze (Fertigspritze), bestehend aus einem Kunststoff-Spritzenkörper (1) mit einem Kunststoff-Spritzenzylinder (2), an dessen rückwärtigem Ende eine Fingerauflage (4) angebracht ist, sowie an dessen austrittsseitigem Kopfende ein Spritzenkopf (5) mit einem Adapterkonus (6) oder einer verriegelbaren Kegelverbindung (8) zur Aufnahme des Gegenstückes des Applizierelementes angeformt ist, mit den innerhalb der einzigen durchgängigen Fertigungslinie in einer kontrollierten Umgebung ausgeführten Verfahrensschritten:
- Fertigen des Kunststoff-Spritzenkörpers (1) durch Spritzgießen in einer entsprechenden formgebenden Höhlung des Spritzgießwerkzeuges mit Vorgabe des Innenmaßes durch einen Kern,
- Anbringen des Verschlusses (7, 12, 14; 16) an dem Spritzenkopf (5,6,8) des Spritzenkörpers (1),
- Silikonisieren der vom Spritzgießen noch warmen und aktiven Oberfläche der Innenwandung des Kunststoff-Spritzenzylinders (2), alternativ auch vor dem Anbringen des Verschlusses,
- Befüllen des Kunststoff-Spritzenkörpers (1) mit dem Spritzeninhalt über das rückwärtige offene Ende des Kunststoff-Spritzenzylinders (2) und
- Verschließen des rückwärtigen offenen Endes des Kunststoff-Spritzenzylinders (2) mit dem Kolbenstopfen (3).

3. Verfahren zum Herstellen eines Kunststoff-Spritzenkorpus (1) für einen befüllten Kunststoff-Spritzenzylinder (Spritzenampulle), bestehend aus einem Kunststoff-Spritzenzylinder (2) ohne Fingerauflage am rückwärtigen Ende, an dessen austrittsseitigem Kopfende ein Spritzenkopf (5) mit einem Adapterkonus (6) oder einer verriegelbaren Kegelverbindung (8) zur Aufnahme des Gegenstückes des Applizierelementes angeformt ist, mit den innerhalb der einzigen durchgängigen Fertigungslinie in einer kontrollierten Umgebung ausgeführten Verfahrensschritten:
- Fertigen des Kunststoff-Spritzenzylinders (2) durch Spritzgießen in einer entsprechenden formgebenden Höhlung des Spritzgießwerkzeuges mit Vorgabe des Innenmaßes durch einen Kern,
- Anbringen des Verschlusses (7, 12, 14; 16) an dem Spritzenkopf (5,6,8) des Kunststoff-Spritzenzylinders (2),
- Silikonisieren der vom Spritzgießen noch warmen und aktiven Oberfläche der Innenwandung des Kunststoff-Spritzenzylinders (2), alternativ auch vor dem Anbringen des Verschlusses,
- Befüllen des Kunststoff-Spritzenzylinders (2) mit dem Spritzeninhalt über das rückwärtige offene Ende des Kunststoff-Spritzenzylinders (2) und
- Verschließen des rückwärtigen offenen Endes des Kunststoff-Spritzenzylinders (2) mit dem Kolbenstopfen (3).

4. Verfahren zum Herstellen eines Kunststoff-Spritzenkorpus (1) für eine befüllte Karpule, bestehend aus einem Kunststoff-Karpulen-Zylinder (17) als Spritzenzylinder, der rückwärtig mit einem Kolbenstopfen (3) verschlossen ist, und der austrittsseitig ein angeformtes Halsteil (19) mit flachem Mündungsrand und einem Randwulst (20) aufweist, das mittels einer Aluminium-Bördelkappe (22) mit eingelegter Dichtscheibe (18) dicht verschlossen ist, mit den innerhalb der einzigen durchgängigen Fertigungslinie in einer kontrollierten Umgebung ausgeführten Verfahrensschritten:
- Fertigen des Kunststoff-Karpulen-Zylinders (17) durch Spritzgießen in einer entsprechenden formgebenden Höhlung des Spritzgießwerkzeuges mit Vorgabe des Innenmaßes durch einen Kern,
- Verschließen des Halsteiles (19) mit der von außen zugeführten Aluminium-Bördelkappe (22) einschließlich Dichtscheibe (18),
- Silikonisieren der vom Spritzgießen noch warmen und aktiven Oberfläche der Innenwandung des Kunststoff-Karpulen-Zylinders (17), alternativ auch vor dem Aufbringen der Aluminium-Bördelkappe mit Dichtscheibe,
- Befüllen des Kunststoff-Karpulen-Zylinders (17) mit dem Spritzeninhalt über das rückwärtige offene Ende des Kunststoff-Karpulen-Zylinders (17),
- Verschließen des rückwärtigen Endes mit dem Kolbenstopfen (3).

5. Verfahren nach einem der Ansprüche 2-4, **dadurch gekennzeichnet, daß** zunächst das rückwärtige Ende des Kunststoff-Spritzen- bzw. Kunststoff-Karpulen-Zylinders (2,17) verschlossen, danach dieser Zylinder über das austrittsseitige Kopfende (5;19) gefüllt und anschließend dieses Kopfende verschlossen wird.

6. Verfahren nach mindestens einem der Ansprüche 1 bis 5, **dadurch gekennzeichnet, daß** die Verfahrensschritte in einer als Sterilraum ausgebildeten kontrollierten Umgebung durchgeführt werden.

7. Verfahren nach mindestens einem der Ansprüche 1 bis 5, **dadurch gekennzeichnet, daß** der befüllte und verschlossene Kunststoff-Spritzenkorpus (1) sterilisiert wird.

8. Verfahren nach Anspruch 7, **dadurch gekennzeichnet, daß** das Sterilisieren durch Autoklavieren erfolgt.

9. Verfahren nach Anspruch 7, **dadurch gekennzeichnet, daß** das Sterilisieren durch energiereiche Strahlung, insbesondere GammaStrahlen, erfolgt.

10. Verfahren nach einem der Ansprüche 1-9, **dadurch gekennzeichnet, daß** der steril befüllte und verschlossene Kunststoff-Spritzenkorpus (1) etikettiert wird und eine weitere Konfektionierung erfährt.

11. Verfahren nach Anspruch 2 oder 3 oder einem der Ansprüche 4-10, **dadurch gekennzeichnet, daß** das Anbringen des austrittsseitigen Verschlusses durch Zufuhr und Aufsetzen oder durch Aufspritzen einer Verschlußkappe aus weichelastischem Material (Tip-Cap) erfolgt.

12. Verfahren nach Anspruch 2 oder 3 oder einem der Ansprüche 4-10, **dadurch gekennzeichnet, daß** das Anbringen des austrittsseitigen Verschlusses durch Anformen eines Knebelverschlusses (7) mit Sollabrißstelle aus elastischem oder weichem Kunststoff-Material im Wege des Spritzgießens erfolgt.

13. Verfahren nach Anspruch 12, **dadurch gekennzeichnet, daß** das Spritzgießen des Knebelverschlusses (7) unter Zufuhr eines unterschiedlichen Kunststoff-Materials in derselben Spritzgußform erfolgt, in der der Kunststoff-Spritzenkorpus (1) geformt wird.

14. Verfahren nach Anspruch 2 oder 3 oder einem der Ansprüche 4-10, **dadurch gekennzeichnet, daß** das Anbringen des austrittsseitigen Verschlusses durch Anformen eines Knebelverschlusses in Form eines Verschlußnippels (12) aus hartem Kunststoffmaterial mit Sollbruchstelle (13) im Wege des Spritzgießens erfolgt, über den anschließend eine Schutzkappe (14) aus einem weichelastischem Kunststoff angespritzt wird.

15. Verfahren nach Anspruch 2 oder 3 oder einem der Ansprüche 4-10, **dadurch gekennzeichnet, daß** das Anbringen des austrittsseitigen Verschlusses durch direktes formschlüssiges Verschließen der Austrittsöffnung (15) mittels eines Pfropfens (16) erfolgt.

16. Verfahren nach mindestens einem der Ansprüche 1 bis 15, **dadurch gekennzeichnet, daß** das Verschließen des rückwärtigen offenen Endes des Kunststoff-Spritzenzylinders (2) bzw. des Kunststoff-Karpul-Zylinders (17) unter Zufuhr des Kolbenstopfens (3) erfolgt.

17. Verfahren nach mindestens einem der Ansprüche 1 bis 15, **dadurch gekennzeichnet, daß** das Verschließen des rückwärtigen offenen Endes des Kunststoff-Spritzenzylinders (2) bzw. des Kunststoff-Karpul-Zylinders (17) unter Spritzgießen des Kolbenstopfens (3) unmittelbar in den Zylinder erfolgt.

18. Verfahren nach einem der Ansprüche 1-17, **dadurch gekennzeichnet, daß** der Spritzeninhalt ein in der Pharmazie zum Auflösen pulverförmiger Arzneimittel oder Diagnostika übliches Lösungsmittel ist.

19. Verfahren nach Anspruch 18, **dadurch gekennzeichnet, daß** das Lösungsmittel Wasser für Injektionszwecke ist.

20. Verfahren nach Anspruch 18, **dadurch gekennzeichnet, daß** das Lösungsmittel eine 0,3 %-ige Natriumchloridlösung ist.

21. Verfahren nach Anspruch 18, **dadurch gekennzeichnet, daß** der Spritzeninhalt ein Lokalanästetikum für die Zahnmedizin ist.

22. Verfahren nach einem der Ansprüche 1-17, **dadurch gekennzeichnet, daß** der Spritzeninhalt ein insulinhaltiges Präparat ist.

## Claims

1. Method of producing a filled plastic syringe body (1) for medical purposes, consisting of a plastic syringe barrel (2, 17) at one end of which, the rear end, a plunger stopper (3) is inserted so as to seal it, and on the other end of which, the outlet end, a head (5, 6, 8, 19, 20) is integrally moulded, which head is designed to receive an application element and, in the storage state, is sealed in a leaktight manner by means of a sealing element (7; 12, 14, 16; 22), said method comprising the following method steps performed within a single continuous manufacturing line in a controlled environment (12):
- forming the plastic syringe body (1) by injection-moulding in a suitable shaping cavity of the injection-mould tool, the inside dimensions being preset by a core,
- sealing one end of the plastic syringe barrel (2, 17),
- siliconizing the surface of the inner wall of the plastic syringe barrel, which surface is still warm and active from the injection-moulding, alternatively also before its one end is sealed,
- filling the plastic syringe body (1) with the syringe content via the other, open end of the plastic syringe barrel;
- sealing the other end of the plastic syringe barrel (2, 17).

2. Method of producing a plastic syringe body for a filled disposable syringe (ready-to-use syringe), consisting of a plastic syringe body (1) with a plastic syringe barrel (2) at the rear end of which a finger rest (4) is arranged, and on the outlet head end of which a syringe head (5) is integrally moulded with an adapter cone (6) or a lockable conical connection (8) for receiving the mating piece of the application element, said method comprising the following method steps performed within the single continuous manufacturing line in a controlled environment:
- forming the plastic syringe body (1) by injection-moulding in a suitable shaping cavity of the injection-mould tool, the inside dimensions being preset by a core,
- applying the seal (7, 12, 14; 16) on the syringe head (5, 6, 8) of the syringe body (1),
- siliconizing the surface of the inner wall of the plastic syringe barrel (2), which surface is still warm and active from the injection-moulding, alternatively also before application of the seal,
- filling the plastic syringe body (1) with the syringe content via the rear, open end of the plastic syringe barrel (2), and
- sealing the rear, open end of the plastic syringe barrel (2) with the plunger stopper (3).

3. Method of producing a plastic syringe body (1) for a filled plastic syringe barrel (syringe ampule), consisting of a plastic syringe barrel (2) without a finger rest at the rear end, and on the outlet head end of which a syringe head (5) is integrally moulded with an adapter cone (6) or a lockable conical connection (8) for receiving the mating piece of the application element, said method comprising the following method steps performed within the single continuous manufacturing line in a controlled environment:
- forming the plastic syringe barrel (2) by injection-moulding in a suitable shaping cavity of the injection-mould tool, the inside dimensions being preset by a core,
- applying the seal (7, 12, 14; 16) on the syringe head (5, 6, 8) of the plastic syringe barrel (2),
- siliconizing the surface of the inner wall of the plastic syringe barrel (2), which surface is still warm and active from the injection-moulding, alternatively also before application of the seal,
- filling the plastic syringe barrel (2) with the syringe content via the rear, open end of the plastic syringe barrel (2), and
- sealing the rear, open end of the plastic syringe barrel (2) with the plunger stopper (3).

4. Method of producing a plastic syringe body (1) for a filled carpule, consisting of a plastic carpule barrel (17) as syringe barrel which at the rear end is sealed with a plunger stopper (3) and which at the outlet end has a moulded-on neck part (19) with a flat mouth edge and an edge bead (20) which is sealed in a leaktight manner by means of an aluminium flanged cap (22) with inserted washer (18), said method comprising the following method steps performed within the single continuous manufacturing line in a controlled environment:
- forming the plastic carpule barrel (17) by injection-moulding in a suitable shaping cavity of the injection-mould tool, the inside dimensions being preset by a core,
- sealing the neck part (19) with the aluminium flanged cap (22), advanced from outside, together with washer (18),
- siliconizing the surface of the inner wall of the plastic carpule barrel (17), which surface is still warm and active from the injection-moulding, alternatively also before application of the aluminium flanged cap with washer,
- filling the plastic carpule barrel (17) with the syringe content via the rear, open end of the plastic carpule barrel (17), and
- sealing the rear end with the plunger stopper (3).

5. Method according to one of Claims 2-4, **characterized in that** the rear end of the plastic syringe barrel (2) or plastic carpule barrel (17) is first sealed, after which this barrel is filled via the outlet head end (5; 19) and this head end is then sealed.

6. Method according to at least one of Claims 1 to 5, **characterized in that** the method steps are carried out in a controlled environment designed as a sterile room.

7. Method according to at least one of Claims 1 to 5, **characterized in that** the filled and sealed plastic syringe body (1) is sterilized.

8. Method according to Claim 7, **characterized in that** the sterilization is effected by autoclaving.

9. Method according to Claim 7, **characterized in that** the sterilization is effected by high-energy radiation, in particular gamma rays.

10. Method according to one of Claims 1-9, **characterized in that** the plastic syringe body (1) filled and sealed under sterile conditions is labelled and undergoes further finishing.

11. Method according to Claim 2 or 3 or one of Claims 4-10, **characterized in that** the seal at the outlet end is obtained by advancing and attaching, or by moulding-on, of a sealing cap made of soft elastic material (tip cap).

12. Method according to Claim 2 or 3 or one of Claims 4-10, **characterized in that** the seal at the outlet end is obtained by integrally moulding a toggle closure (7) with a predetermined breaking point made of elastic or soft plastic material during the injection-moulding.

13. Method according to Claim 12, **characterized in that** the toggle closure (7) is injection-moulded with supply of a different plastic material in the same injection-mould tool in which the plastic syringe body (1) is moulded.

14. Method according to Claim 2 or 3 or one of Claims 4-10, **characterized in that** the seal at the outlet end is applied by integrally moulding a toggle closure in the form of a sealing nipple (12) made of hard plastic material with a predetermined breaking point (13) during the injection-moulding, over which sealing nipple (12) a protective cap (14) made of a soft elastic plastic is then moulded.

15. Method according to Claim 2 or 3 or one of Claims 4-10, **characterized in that** the seal at the outlet end is applied by direct form-fitting closure of the outlet opening (15) by means of a plug (16).

16. Method according to at least one of Claims 1 to 15, **characterized in that** the rear, open end of the plastic syringe barrel (2) or of the plastic carpule barrel (17) is sealed by advancing of the plunger stopper (3).

17. Method according to at least one of Claims 1 to 15, **characterized in that** the rear, open end of the plastic syringe barrel (2) or of the plastic carpule barrel (17) is sealed by injection-moulding of the plunger stopper (3) directly in the barrel.

18. Method according to one of Claims 1-17, **characterized in that** the syringe content is a solvent customarily used in the pharmaceutical industry to dissolve powdered pharmaceuticals or diagnostic agents.

19. Method according to Claim 18, **characterized in that** the solvent is water for injection purposes.

20. Method according to Claim 18, **characterized in that** the solvent is a 0.3% strength sodium chloride solution.

21. Method according to Claim 18, **characterized in that** the syringe content is a local anaesthetic for dentistry.

22. Method according to one of Claims 1-17, **characterized in that** the syringe content is an insulin-containing preparation.

## Revendications

1. Procédé de fabrication d'un corps (1) rempli de seringue en matériau synthétique, destiné à des fins médicales, constitué d'un cylindre (2, 17) de seringue en matériau synthétique, un bouchon (3) de piston étant introduit dans son extrémité postérieure, en la fermant et une tête (5, 6, 8, 19, 20) étant moulée en son autre extrémité, du côté de la sortie, qui est exécutée pour recevoir un élément d'application et qui est fermée de manière étanche au moyen d'un élément de fermeture (7 ; 12, 14, 16 ; 22) dans l'état d'entreposage, comprenant les étapes de procédé réalisées dans une seule ligne de fabrication continue dans un environnement (12) contrôlé :
- fabrication du corps (1) de seringue en matériau synthétique par moulage par injection dans un creux moulant correspondant du moule à injection avec définition de la taille intérieure par un noyau,
- fermeture d'une première extrémité du cylindre (2, 17) de la seringue en matériau synthétique,
- revêtement avec du silicone de la surface de la paroi intérieure du cylindre de seringue en matériau synthétique, encore chaude et active suite au moulage par injection, de manière alternative également avant la fermeture de sa première extrémité,
- remplissage du corps (1) de seringue en matériau synthétique avec le contenu de la seringue via la seconde extrémité ouverte du cylindre de seringue en matériau synthétique,
- fermeture de la seconde extrémité du cylindre (2, 17) de seringue en matériau synthétique.

2. Procédé de fabrication d'un corps de seringue en matériau synthétique pour une seringue à usage unique remplie (seringue prête à l'emploi), constituée d'un corps (1) de seringue en matériau synthétique avec un cylindre (2) de seringue en matériau synthétique, un emplacement (4) pour un doigt étant réalisé en son extrémité postérieure et une tête (5) de seringue étant moulée en son extrémité de tête du côté de la sortie, avec un cône d'adaptation (6) ou un raccord conique (8) verrouillable destiné à recevoir la contre-dépouille de l'élément d'application, comprenant les étapes de procédé réalisées dans la seule ligne de fabrication continue dans un environnement contrôlé :
- fabrication du corps (1) de seringue en matériau synthétique par moulage par injection dans un creux moulant correspondant du moule à injection avec définition de la taille intérieure par un noyau,
- mise en place de la fermeture (7, 12, 14 ; 16) sur la tête (5, 6, 8) de la seringue du corps (1) de seringue,
- revêtement avec du silicone de la surface de la paroi intérieure du cylindre (2) de seringue en matériau synthétique, encore chaude et active suite au moulage par injection, de manière alternative également avant la mise en place de la fermeture,
- remplissage du corps (1) de seringue en matériau synthétique avec le contenu de la seringue via l'extrémité postérieure ouverte du cylindre (2) de seringue en matériau synthétique, et
- fermeture de l'extrémité postérieure ouverte du cylindre (2) de seringue en matériau synthétique par le bouchon (3) du piston.

3. Procédé de fabrication d'un corps (1) de seringue en matériau synthétique pour un cylindre rempli de seringue en matériau synthétique (ampoule de seringue), constitué d'un cylindre (2) de seringue en matériau synthétique sans emplacement pour le doigt en l'extrémité postérieure, une tête (5) de seringue étant moulée en son extrémité de tête du côté de la sortie, avec un cône d'adaptation (6) ou un raccord conique (8) verrouillable destiné à recevoir la contre-dépouille de l'élément d'application, comprenant les étapes de procédé réalisées dans la seule ligne de fabrication continue dans un environnement contrôlé :
- fabrication du cylindre (2) de seringue en matériau synthétique par moulage par injection dans un creux moulant correspondant du moule à injection avec définition de la taille intérieure par un noyau,
- mise en place de la fermeture (7, 12, 14 ; 16) sur la tête (5, 6, 8) de la seringue du cylindre (2) de seringue en matériau synthétique,
- revêtement avec du silicone de la surface de la paroi intérieure du cylindre (2) de seringue en matériau synthétique, encore chaude et active suite au moulage par injection, de manière alternative également avant la mise en place de la fermeture,
- remplissage du cylindre (2) de seringue en matériau synthétique avec le contenu de la seringue via l'extrémité postérieure ouverte du cylindre (2) de seringue en matériau synthétique, et
- fermeture de l'extrémité postérieure ouverte du cylindre (2) de seringue en matériau synthétique par le bouchon (3) du piston.

4. Procédé de fabrication d'un corps (1) de seringue en matériau synthétique pour une carpule remplie, constituée d'un cylindre (17) de carpule en matériau synthétique comme cylindre de seringue, qui est fermé en l'extrémité postérieure par un bouchon (3) de piston et qui présente du côté de la sortie une partie de col (19) moulée avec un bord d'orifice plat et un bourrelet (20) de bord qui est fermée de manière étanche au moyen d'un capuchon serti (22) en aluminium dans lequel est placée une garniture d'étanchéité (18), comprenant les étapes de procédé réalisées dans la seule ligne de fabrication continue dans un environnement contrôlé :
- fabrication du cylindre (17) de carpule en matériau synthétique par moulage par injection dans un creux moulant correspondant du moule à injection avec définition de la taille intérieure par un noyau,
- fermeture de la partie de col (19) avec le capuchon serti (22) en aluminium, comprenant la garniture d'étanchéité (18), placé depuis l'extérieur,
- revêtement avec du silicone de la surface de la paroi intérieure du cylindre (17) de carpule en matériau synthétique, encore chaude et active suite au moulage par injection, de manière alternative également avant la mise en place du capuchon serti en aluminium avec la garniture d'étanchéité,
- remplissage du cylindre (17) de carpule en matériau synthétique avec le contenu de la seringue via l'extrémité postérieure ouverte du cylindre (17) de carpule en matériau synthétique,
- fermeture de l'extrémité postérieure par le bouchon (3) du piston.

5. Procédé selon l'une quelconque des revendications 2 à 4, **caractérisé en ce qu'**on ferme d'abord l'extrémité postérieure du cylindre (2, 17) de seringue ou, selon le cas, de carpule en matériau synthétique, ensuite on remplit ce cylindre via l'extrémité (5 ; 19) de tête du côté de la sortie puis on ferme cette extrémité de tête.

6. Procédé selon au moins l'une quelconque des revendications 1 à 5, **caractérisé en ce que** les étapes de procédé sont réalisées dans un environnement contrôlé exécuté sous forme d'une chambre stérile.

7. Procédé selon au moins l'une quelconque des revendications 1 à 5, **caractérisé en ce que** le corps (1) de seringue en matériau synthétique rempli et fermé est stérilisé.

8. Procédé selon la revendication 7, **caractérisé en ce que** la stérilisation est réalisée dans un autoclave.

9. Procédé selon la revendication 7, **caractérisé en ce que** la stérilisation est réalisée par un rayonnement riche en énergie, en particulier des rayons gamma.

10. Procédé selon l'une quelconque des revendications 1 à 9, **caractérisé en ce que** le corps (1) de seringue en matériau synthétique rempli et fermé de manière stérile est étiqueté et subit une confection ultérieure.

11. Procédé selon la revendication 2 ou 3 ou l'une quelconque des revendications 4 à 10, **caractérisé en ce que** la mise en place de la fermeture du côté de la sortie est réalisée par l'apport et le placement ou par moulage d'un capuchon de fermeture en matériau élastomère (Tip-Cap).

12. Procédé selon la revendication 2 ou 3 ou l'une quelconque des revendications 4 à 10, **caractérisé en ce que** la mise en place de la fermeture du côté de la sortie est réalisée par moulage d'un raccord à cordon (7) avec une zone de rupture intentionnellement prévue en matériau synthétique élastique ou souple au cours du moulage par injection.

13. Procédé selon la revendication 12, **caractérisé en ce que** le moulage par injection du raccord à cordon (7) est réalisé en introduisant un matériau synthétique différent dans le même moule à injection que celui dans lequel est moulé le corps (1) de seringue en matériau synthétique.

14. Procédé selon la revendication 2 ou 3 ou l'une quelconque des revendications 4 à 10, **caractérisé en ce que** la mise en place de la fermeture du côté de la sortie est réalisée par moulage d'un raccord à cordon sous forme d'un raccord fileté (12) de fermeture en matériau synthétique rigide avec une zone de rupture intentionnellement prévue (13) au cours du moulage par injection, sur lequel est ensuite moulé un capuchon de protection (14) en matériau synthétique élastomère.

15. Procédé selon la revendication 2 ou 3 ou l'une quelconque des revendications 4 à 10, **caractérisé en ce que** la mise en place de la fermeture du côté de la sortie est réalisée par fermeture géométrique directe de l'ouverture de sortie (15) au moyen d'un bouchon (16).

16. Procédé selon au moins l'une quelconque des revendications 1 à 15, **caractérisé en ce que** la fermeture de l'ouverture postérieure ouverte du cylindre (2) de seringue en matériau synthétique ou, selon le cas, du cylindre (17) de carpule en matériau synthétique est réalisée en introduisant le bouchon (3) du piston.

17. Procédé selon au moins l'une quelconque des revendications 1 à 15, **caractérisé en ce que** la fermeture de l'ouverture postérieure ouverte du cylindre (2) de seringue en matériau synthétique ou, selon le cas, du cylindre (17) de carpule en matériau synthétique est réalisée par moulage par injection du bouchon (3) du piston directement dans le cylindre.

18. Procédé selon l'une quelconque des revendications 1 à 17, **caractérisé en ce que** le contenu de la seringue est un solvant usuel en pharmacie pour la dissolution d'un médicament ou d'un produit diagnostique sous forme de poudre.

19. Procédé selon la revendication 18, **caractérisé en ce que** le solvant est de l'eau destinée à l'injection.

20. Procédé selon la revendication 18, **caractérisé en ce que** le solvant est une solution à 0,3% de chlorure de sodium.

21. Procédé selon la revendication 18, **caractérisé en ce que** le contenu de la seringue est un anesthésique local pour la médecine dentaire.

22. Procédé selon l'une quelconque des revendications 1 à 17, **caractérisé en ce que** le contenu de la seringue est une préparation contenant de l'insuline.
